# EUROPEAN PATENT SPECIFICATION

(11) **EP 3 880 154 B1**
(45) Date of publication and mention of the grant of the patent: **23.10.2024**
(21) Application number: 19885631.2
(22) Date of filing: 15.11.2019
(51) Int. Cl.: A61J 1/20, A61J 1/14

(54) **FLUID TRANSFER DEVICE**
FLÜSSIGTRANSFERVORRICHTUNG
DISPOSITIF DE TRANSFERT DE FLUIDE

(30) Priority: 16.11.2018 CA 3024462
(43) Date of publication of application: 22.09.2021
(73) Proprietor: Septodont Holding, 94100 Saint-Maur-Des-Fossés (FR)
(72) Inventor: HAMEL, Simon, Quebec J0E 1V0 (CA); LAFONTAINE LACASSE, Marie, Sherbrooke, Quebec J1G 2L3 (CA)
(74) Representative: Icosa
(86) International application number: PCT/CA2019/000157
(87) International publication number: WO 2020/097715

(56) References cited:
- WO-A1-2008/151431
- WO-A1-2010/078651
- WO-A1-2011/050468
- WO-A1-2013/134246
- WO-A1-2013/134246
- WO-A2-2013/134614
- US-A1- 2005 124 964
- US-A1- 2009 292 271
- US-A1- 2010 298 768
- US-A1- 2010 298 768
- US-A1- 2011 166 543
- US-A1- 2014 311 624
- US-A1- 2015 082 746

## Description

### FIELD OF THE INVENTION

The present invention relates to fluid transfer and more particularly, relates to an apparatus for transferring fluid from a first container to a fluid filled second container.

### BACKGROUND OF THE INVENTION

The transfer of a liquid or fluid from one container to another is well known in the art and many devices are utilized for facilitating the same. Such devices are known from US 2010/298768 A1 and WO 2013/134246 A1. However, in the medical field. substantial care must be utilized in doing so while complying with all the requirements for sterility and the like. One particular problem arises when it is desired to transfer liquid from a first container into a second container and wherein the second container already contains a liquid. The second container is already filled with a liquid and such a container typically will comprise a syringe body which is a glass cylinder having a plunger at one end and a stopper or sealing member at the second end. A needle may optionally be attached. Normally the body has been sterilized along with the plunger and it would not be acceptable procedure to allow the plunger to move into a non sterile area, as would happen if more fluids were placed in the container. As used herein, the term vial refers to a glass container with a pierceable stopper while a cartridge is a glass container with a pierceable stopper and a plunger.

Transfer of such fluids between containers is required under certain circumstances. An example of one such set of circumstances is when a liquid is utilized for freezing flesh (rendering the flesh insensitive to pain). A typical injection for freezing requires that the liquid to be injected to be acidic. The acidic nature of the liquid subsequently causes pain to the patient. Accordingly, it is desirable to neutralize or buffer the liquid using a compound such as sodium bicarbonate.

### SUMMARY OF THE INVENTION

According to the present invention, there is provided a device for combining two liquids whereby a portion of a first liquid is permitted to discharge into a storage portion of the device. The invention is defined by the appended claims.

### BRIEF DESCRIPTION OF THE DRAWINGS

Having thus generally described the invention, reference will be made to the accompanying drawings illustrating an embodiment thereof, in which:

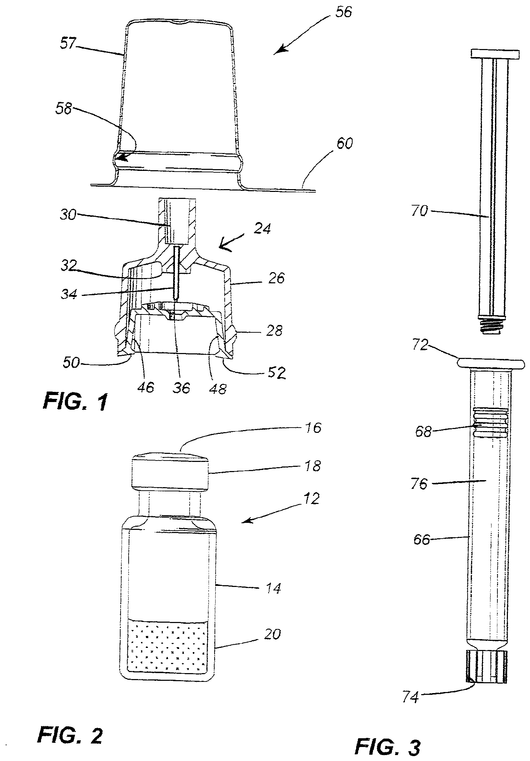

Figure 1 is a side elevational view of a transfer device;
Figure 2 is a side elevational view, partially in section, of a vial containing a medicant;
Figure 3 is a side elevational view of a syringe and plunger rod;

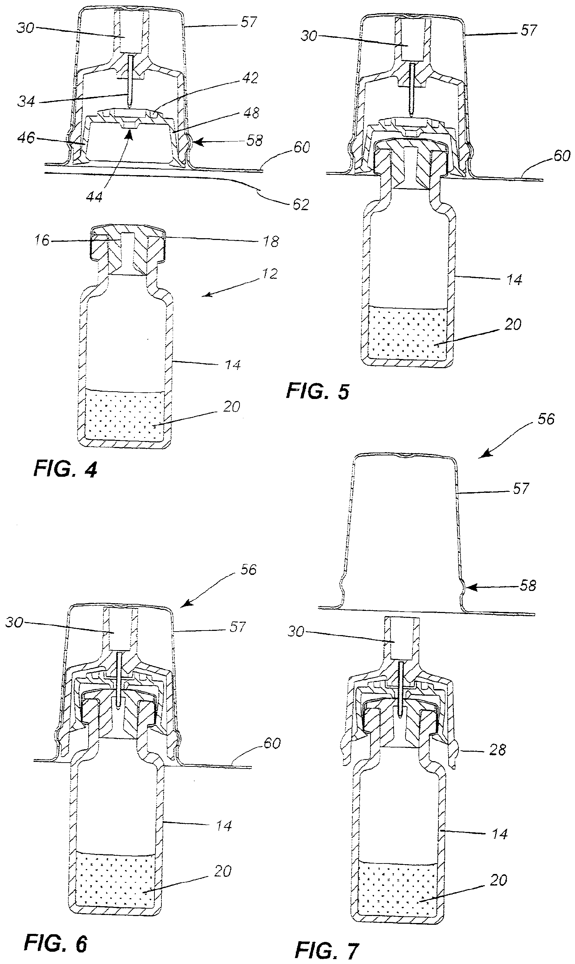

Figure 4 is a cross sectional view of the transfer device prior to its use;
Figure 5 is a side sectional view of the device being placed on a vial;
Figure 6 is a side sectional view showing piercing of the vial;
Figure 7 is a cross sectional view illustrating the cap being removed;
Figure 8 is a view, partially in cross section, of a syringe being attached to the transfer device;
Figure 9 is a cross sectional view illustrating a syringe being ready to be placed on the vial;
Figure 10 is a cross sectional view illustrating the syringe attached to the vial;
Figure 11 is a sectional view illustrating the mixing of components;
Figure 12 is a sectional view illustrating the aspiration of the mixture into the syringe;

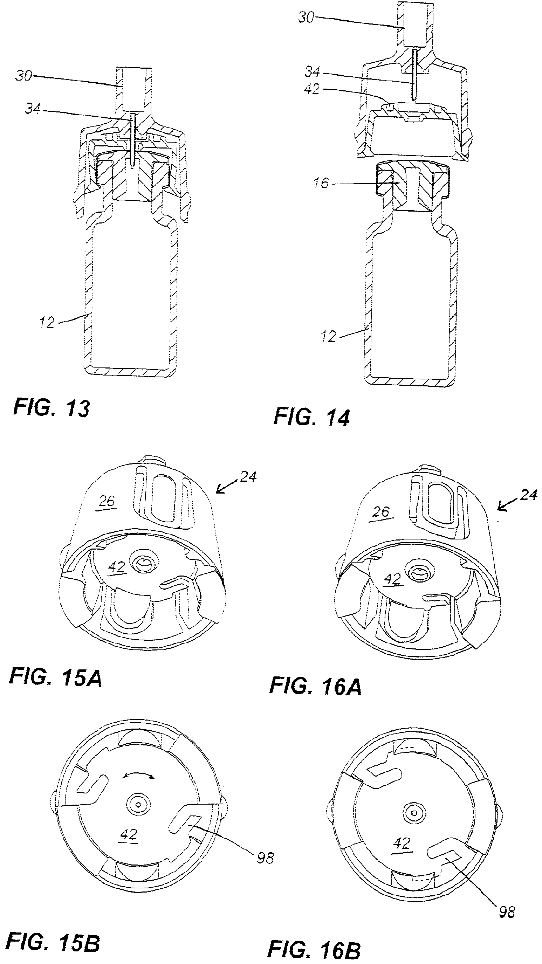

Figure 13 is a cross sectional view illustrating placement of the transfer assembly on a vial;
Figure 14 is an exploded view illustrating the transfer assembly and the vial prior to insertion of the vial;
Figure 15A is a bottom perspective view of a transfer assembly;
Figure 15B is a bottom plan view thereof;
Figure 16A is a perspective view of the transfer assembly according to a further embodiment;
Figure 16B is a bottom plan view thereof;
Figure 17A is an exploded view of the transfer assembly;
Figure 17B is a bottom perspective view thereof;
Figures 17C to 17E show the sequence of placing the transfer assembly on the vial;
Figures 17F to 17H illustrate the placement of the transfer assembly in a further embodiment thereof on a vial;
Figure 18 is an exploded view of the transfer assembly;
Figures 19A to 19D are perspective views illustrating placement of the transfer assembly on a vial and removal thereof;
Figure 20A is a side elevational view of a transfer assembly when packaged;
Figure 20B is an exploded view thereof; and
Figure 21 is a cross sectional view of the needle assembly of the transfer assembly.
Figure 22 is an exploded view of the injector according to an embodiment of the present invention;
Figure 23 illustrates the outer housing and the base member of the injector of Figure 22;
Figure 24 is a side elevational view of the base member;
Figure 25 is a sectional view taken along the lines A-A of Figure 24;
Figure 26 is a side elevational view of a second cartridge and the outer housing;
Figure 27A is a side elevational view of the base member and cartridge inserted therein;
Figure 27B is a sectional view taken along the lines B-B of Figure 27A;
Figure 28A is a sectional view of the lower portion of the injector with a cartridge being inserted therein;
Figure 28B is an enlarged view of the sectional line shown in dashed lines of Figure 28A;
Figure 29 is a sectional view illustrating advancement of the cartridge into the injector;
Figure 30A is a sectional view illustrating piercing of the cartridges;
Figure 30B is an enlarged view of the portion shown in dashed lines of Figure 30A;
Figure 31A illustrates the final step of the piercing with removal of the cartridge therefrom;
Figure 31B is an enlarged view of the portion shown in dashed lines of Figure 31A.

### DETAILED DESCRIPTION OF THE INVENTION

Referring to the drawings in greater detail and by reference characters thereto, there is illustrated a transfer system which is generally designated by reference numeral 10 and which is suitable for use with a vial generally designated by reference numeral 12.

Vial 12 has a body 14 with a neck sealed by a septum 16 over which there is a cap 18. A medicant 20 is contained within body 14 and would typically comprise a dry ingredient although a fluid may also be utilized.

Transfer system 10 includes an outer housing 24 and a circular side wall 26. On circular side wall 26 there is a protrusion 28 near the bottom thereof. On its upper end, there is provided a luer connection 30. An inner wall 32 mounts a needle 34 which is hollow in nature and has a piercing end 36. As previously mentioned, needle 34 may be a spike.

Mounted interiorly of outer housing 24 is a moveable member 40. Moveable member 40 has a top wall 42 with an aperture 44 centrally located therein to permit the passage of needle 34. Extending downwardly from top wall 42 is a first leg 46 and a second leg 48. First leg 46 has an outwardly extending flange 50 at the bottom thereof while second leg 48 also has an outwardly extending flange 52.

A cover 56 is provided to receive transfer system 10. Cover 56 has a side wall 57 with a groove 58 which is adapted to engage with protrusion 28 to retain transfer system 10 in position. Side wall 57 is provided with an outwardly extending flange 60 at the bottom thereof. Flange 60 is designed to receive a peelable sealing strip 62 so as to provide a hermetically sealed package.

The transfer system is preferably utilized with a cartridge which has a syringe body 66 and a plunger 68 mounted therein. A plunger rod 70 is designed to be screwthreadably engageable with plunger 68. Syringe body 66 includes a backstop 72 to permit proper gripping by the hand of a user. At its front end, syringe body 66 includes a luer connector 74. Typically, syringe body 66 is filled with a diluent 76 although any desired fluid may be utilized.

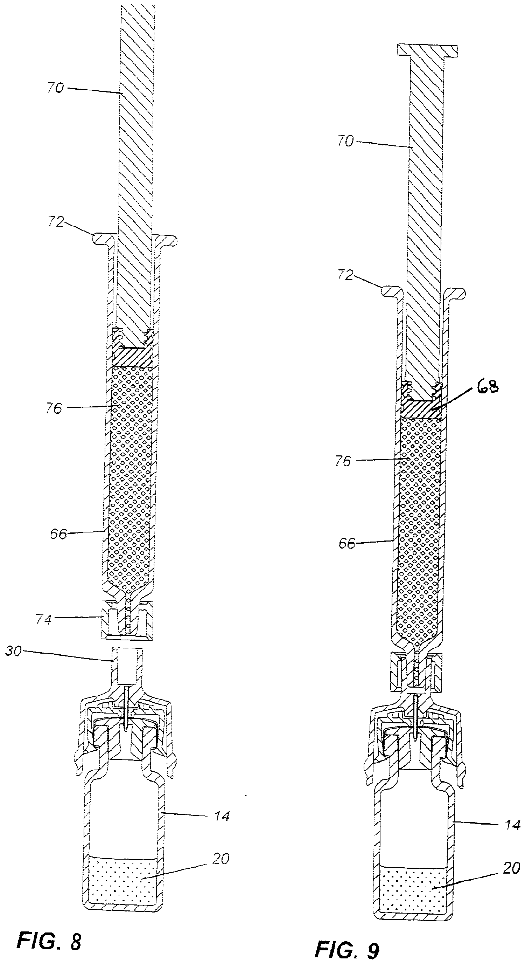

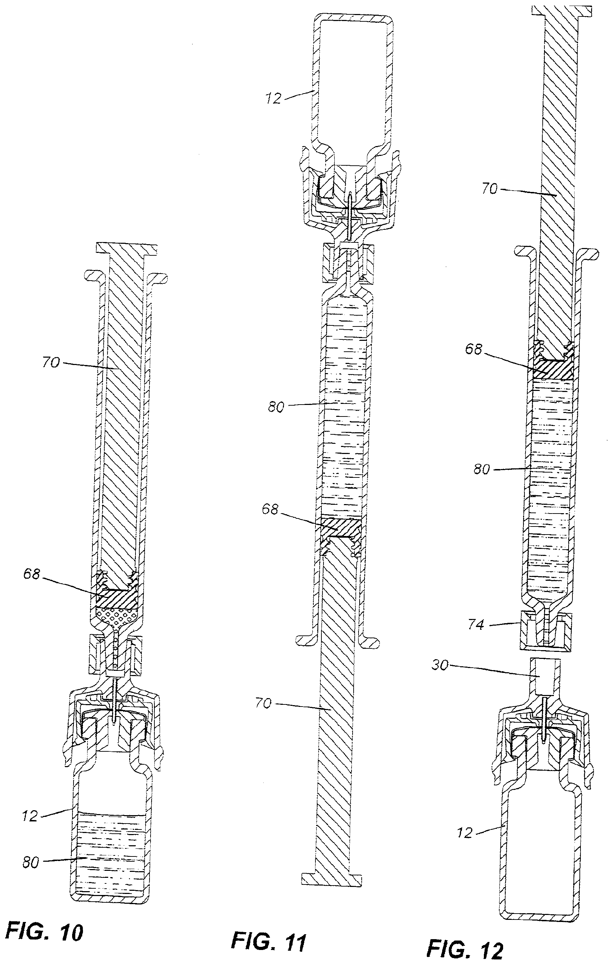

As shown in Figures 8 and 9, plunger rod 70 is connected to plunger 68 and the diluent 76 is then forced into vial body 14 as shown in Figure 10. The medicant and diluent may then be mixed and the assembly inverted as shown in Figure 11. The mixture 80 is then aspirated back into syringe body 66. The mixture 80 is then ready for injection when a needle assembly is connected to luer connector 74.

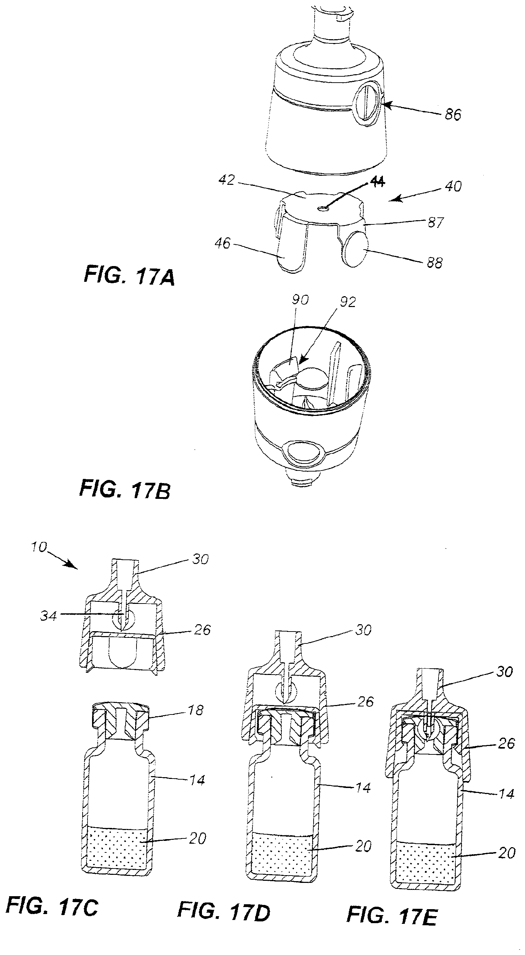

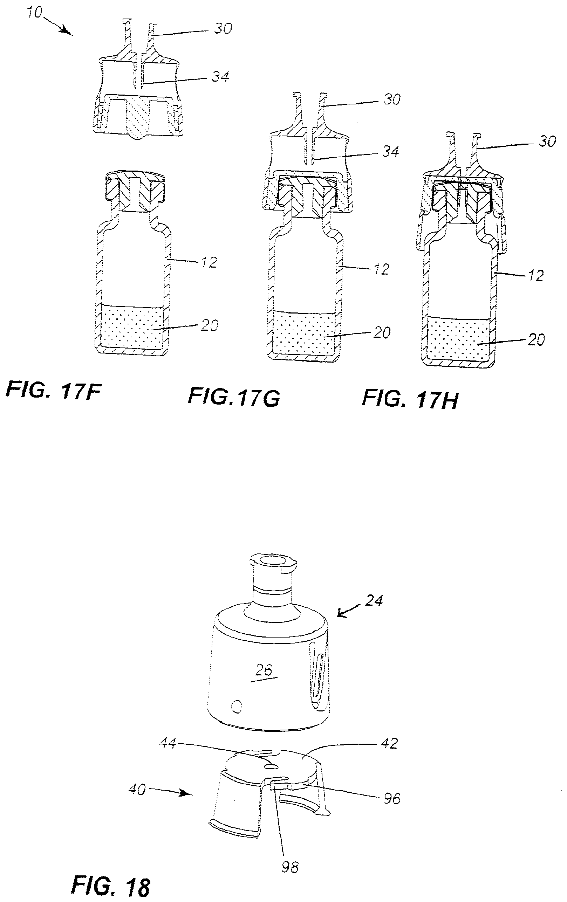

In the embodiment of Figures 17A to 17H, it will be noted that outer housing 24 is provided with a pair of apertures 86 in side wall 26. Also, in this embodiment, there are provided an extra pair of legs 87 each having buttons 88 formed on an exterior surface thereof. In this embodiment, when the moveable member 40 moves upwardly, buttons 88 engage in apertures 86.

On the interior surface of wall 26, there are provided ribs 90 which have a groove 92 formed therein. Thus, when pressure is exerted on buttons 86 as vial 12 is being withdrawn, moveable member 40 will move downwardly until the top wall 42 engages with groove 92. This retains moveable member 40 in position for further use.

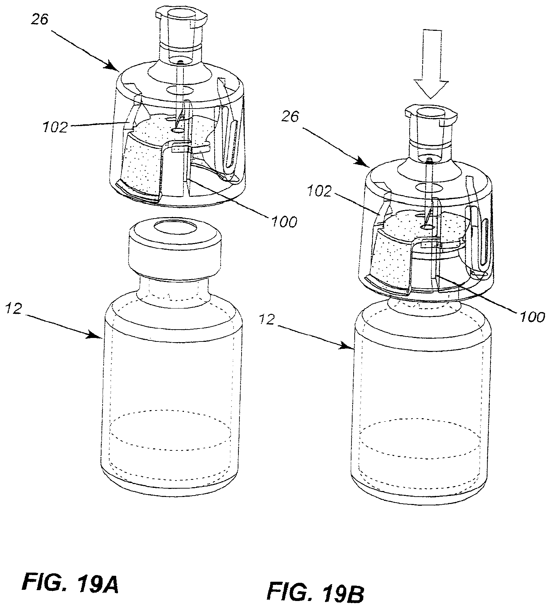

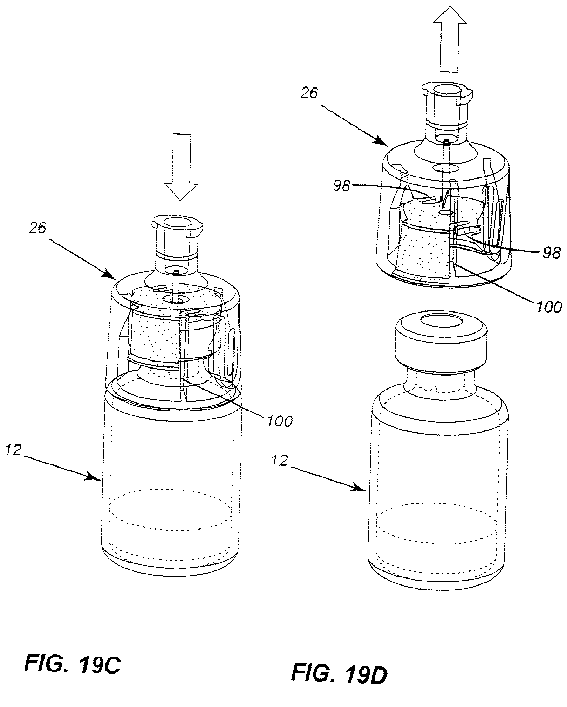

In the embodiment of Figures 18 to 19D, it will be noted that top wall 42 is provided with protrusions 96 and locking latches 98. On the interior there are provided ribs 100 and angled side wall portions 102. The arrangement is such that upon upward movement of moveable member 40, protrusions 96 engage with angled side wall 102 to rotate moveable member 40. Upon withdrawal, locking latches 98 engage with rib 100 so as to prevent further use of the transfer member.

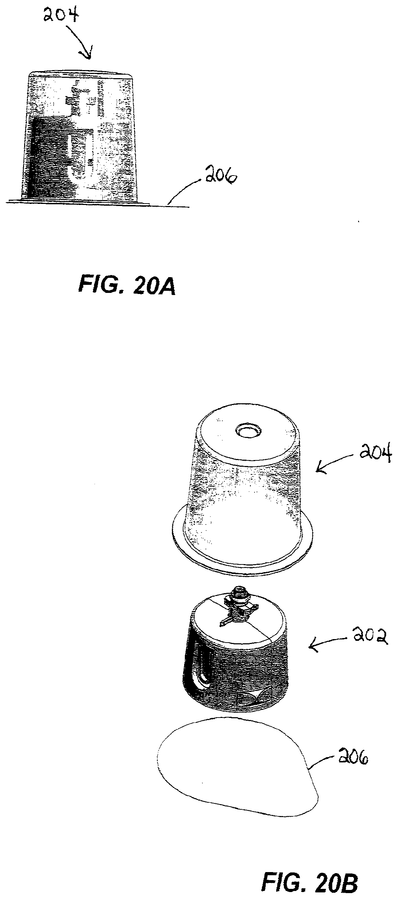

As shown in Figures 20A and 20B, the transfer assembly will comprise the transfer device 202 as previously described herein. A cover 204 is mounted over transfer device 202 and a removable seal 206 applied.

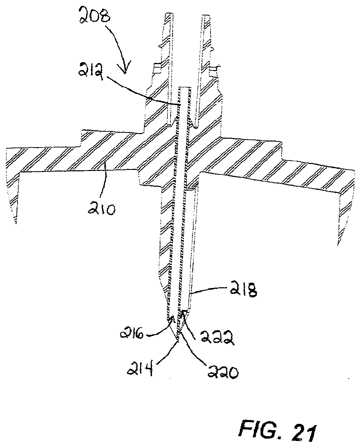

The transfer device includes a needle assembly generally designated by reference numeral 208 and thus seen in Figure 21. A portion of the body 210 has a needle 212 extending therethrough. Needle 212 is of a metallic material and is over molded with body portion 210. Needle 212 includes a piercing tip 214 and a needle passageway 216 extends therethrough. A plastic spike 218 is formed as a portion of needle 212. The plastic spike has a piercing tip 220 with a spike venting passageway 222 formed therein. As may been seen, needle passageway 216 and spike venting passageway 222 lie side by side with a portion of needle 212 forming one wall of spike venting passageway 222. Both needle piercing tip 214 and spike piercing tip 220 are angled to provide the best piercing capability. Both tips are sharpened as is known in the art.

It will be noted that needle passageway 216 extends a little bit further down than the opening to spike venting passageway 222. In use, spike passageway 216 is utilized for transfer of the drug or liquid into a further container, while spike venting passageway 222 permits the egress of either a gas or a liquid therefrom. When transferring from one container to another, a liquid in the second container will exit through spike venting passageway 222 to permit equalization of pressures and to allow the liquid transferred through needle passageway 216 to flow smoothly into the second container.

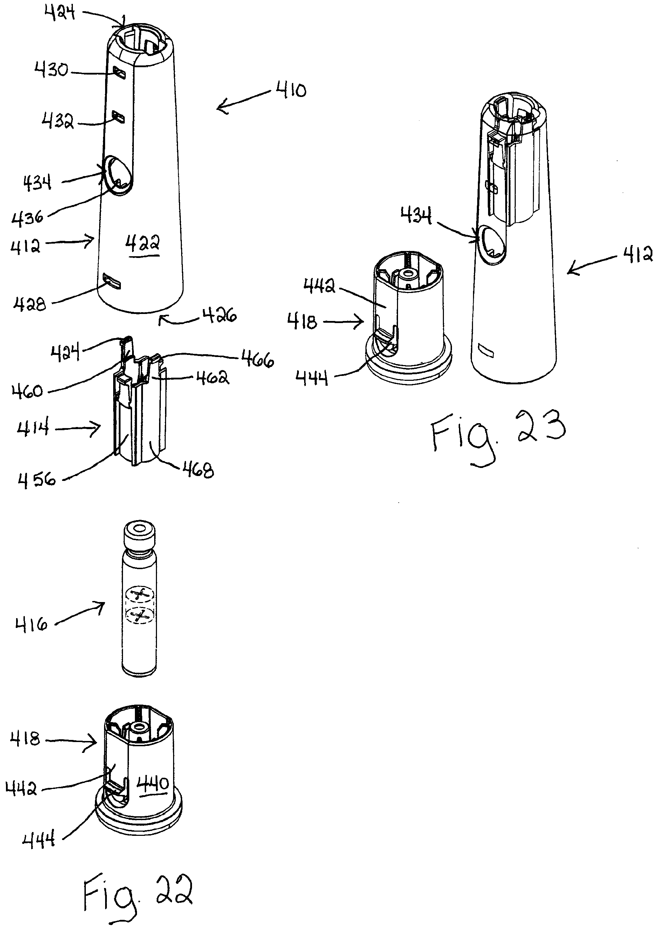

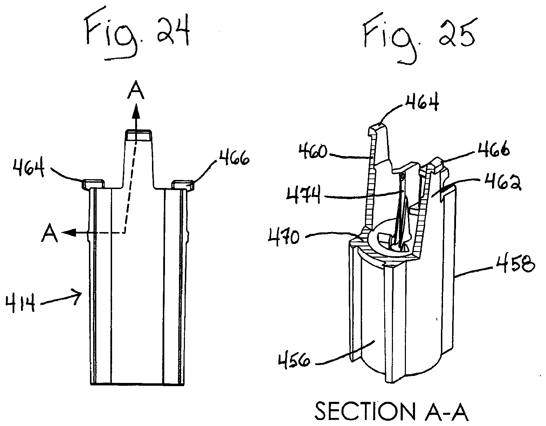

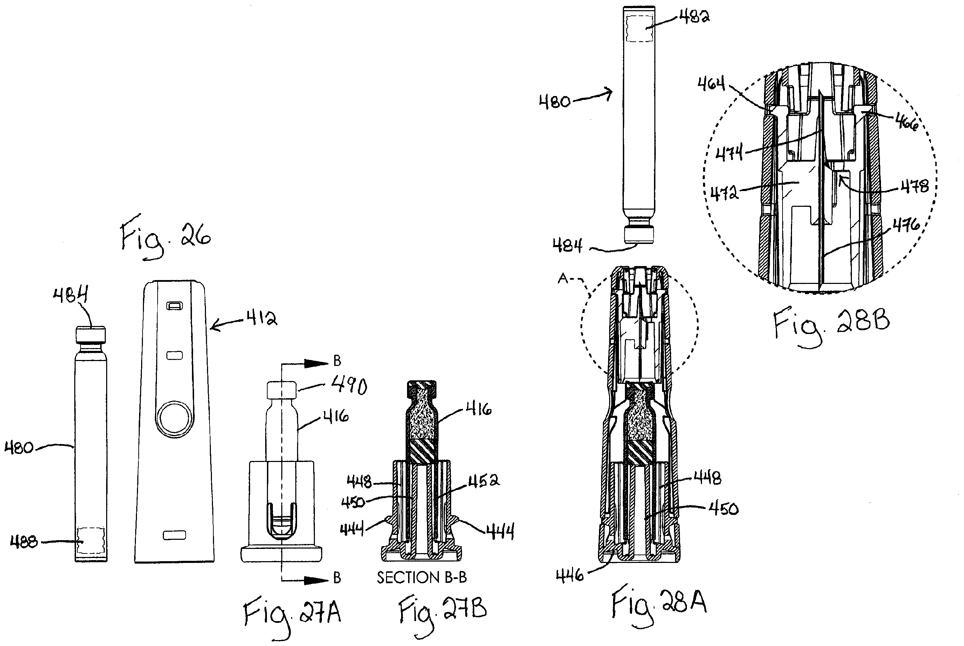

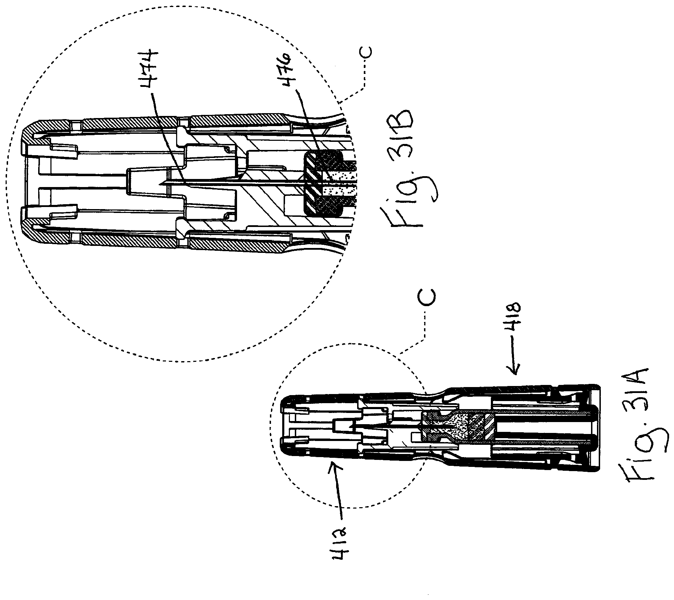

Referring now to the embodiment illustrated in Figures 22 to 31B, there is illustrated a fluid transfer device generally designated by reference numeral 410.

Fluid transfer device 410 includes an outer housing generally designated by reference numeral 412, a needle hub generally designated by reference numeral 414, a cartridge generally designated by reference numeral 416, and a base member generally designated by reference numeral 418.

Outer housing 412 is defined by a slightly oblong wall 422. Outer housing 412 has an open top 424 and an open bottom 426. A pair of lower slots 428 are provided diametrically opposite each other. Located in the upper portion of wall 422 are a pair of diametrically opposed upper slots 430 and a pair of diametrically opposed intermediate slots 432. Wall 422 also has a pair of circular apertures 438 formed in a diametrically opposed pattern. On the interior of wall 422, there are provided diametrically opposed inferior ribs 436.

Base member 418 also has an overall oblong configuration with a side wall 440 and tabs 442, the tabs 442 being diametrically opposed. On each of tabs 442, there is an outwardly extending protrusion 444. Base member 418 also includes a bottom wall 446. Interiorly of side wall 440, there are provided corner ribs 448 with corner ribs being located proximate the corners of base member 418.

Extending upwardly from bottom wall 446 is a plunger rod 450. A pair of guide members 452 are provided, one on each side of plunger rod 450.

Needle hub 414 includes a first side wall 456 and a second side wall 458. Each side wall 456, 458 includes a relatively planar outer edge and an arcuate inner portion between the planar portions. Extending upwardly from side wall 456 is an upper tab 460 while upper tab 462 extends from side wall 458. A protrusion 464 is provided on upper tab 460 and a protrusion 466 is provided on upper tab 462. Needle hub 414 also includes opposed end walls 468, 470. An intermediate wall 472 is provided. Intermediate wall 472 does not extend complete across between end walls 468, 470, and side walls 456, 458 to thereby leave a passageway 478 therein. Mounted on one side of intermediate wall 472 is an upper needle 474 while a lower needle 476 is mounted on the other side of intermediate wall 472.

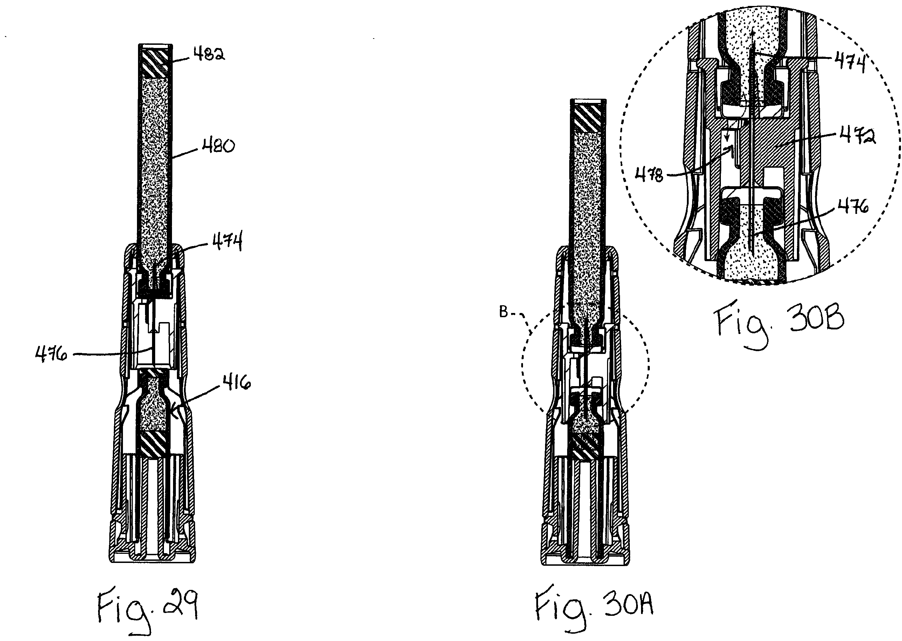

As may be seen in the drawings, first cartridge 416 is placed within base member 418 such that plunger 488 seats on plunger rod 450, while a pierceable cap 490 is facing upwardly. Subsequently, outer housing 412 has first cartridge 416 placed therein and needle hub 414 inserted such that protrusions 464, 466 of upper tabs 460, 462 limit movement to prevent excess movement. Downward pressure on outer housing 412 and first cartridge 416 results in the piercing of pierceable caps 484 and 490 as shown in Figures 29, 30A and 30B. This will result in the transfer of liquid within first cartridge 416 to second cartridge 480. Excess liquid is allowed to flow through passageway 478 due to the venting needle as previously described.

It will be understood that the above described embodiment is for purposes of illustration only and that changes and modifications may be made thereto without departing from the scope of the invention as defined by the appended claims.

## Claims

1. A fluid transfer device (410) comprising:
a housing (412) having an exterior wall (422), a plurality of pairs of slots (428, 430) formed in said exterior wall (422), each one of said pairs of slots being diametrically opposed to each other;
a needle hub (414) having an exterior wall (456, 458) and an interior wall (472) extending interiorly across said exterior wall (456, 458), to thereby form upper and lower compartments (492, 494) a first needle (474) extending upwardly from said interior wall (472), into said upper compartment (492) a second needle (476) extending downwardly from said interior wall into said lower compartment (494), said interior wall (472) having an opening therein to form a fluid passageway (478);
a first cartridge (416) having a plunger and a pierceable cap (490);
a second cartridge (480) having a plunger and a pierceable cap (484);
a base member (418), said member having a base bottom wall (446) and a plunger rod (450); and
said arrangement being such that said first cartridge (416) is inserted into said base member (418) such that said plunger seats on said plunger rod (450), and said second cartridge (480) is in said housing (412) with said pierceable cap (484) of said second cartridge (480) facing said first needle (474);
said fluid transfer device being **characterized in that** the plunger rod (450) is fixedly secured to and extends upwardly from said base bottom wall.

2. The fluid transfer device of Claim 1 wherein said needle hub (414) includes first and second upper tabs (460, 462), each of said upper tabs having a protrusion extending outwardly therefrom, said protrusions being arranged to engage with one of said pairs of slots (428, 430) formed in said housing (412).

3. The fluid transfer device of Claim 1 wherein said first needle has a spike (218) adjacent thereto, said spike (218) and said first needle having a common wall therebetween.

## Patentansprüche

1. Fluidtransfervorrichtung (410), umfassend:
ein Gehäuse (412), das eine Außenwand (422) aufweist, wobei in der Außenwand (422) eine Vielzahl von Schlitzpaaren (428, 430) gebildet sind, wobei jedes der Schlitzpaare einander diametral gegenüberliegt;
eine Nadelnabe (414), die eine Außenwand (456, 458) und eine Innenwand (472) aufweist, die sich innen über die Außenwand (456, 458) hinweg erstreckt, um dadurch ein oberes und ein unteres Fach (492, 494) zu bilden, wobei sich eine erste Nadel (474) von der Innenwand (472) nach oben in das obere Fach (492) erstreckt, wobei sich eine zweite Nadel (476) von der Innenwand nach unten in das untere Fach (494) erstreckt, wobei die Innenwand (472) in sich eine Öffnung aufweist, um einen Fluiddurchgang (478) zu bilden;
eine erste Kartusche (416), die einen Kolben und eine durchstechbare Kappe (490) aufweist;
eine zweite Kartusche (480), die einen Kolben und eine durchstechbare Kappe (484) aufweist;
ein Basiselement (418), wobei das Element eine Basisbodenwand (446) und eine Kolbenstange (450) aufweist; und
wobei die Anordnung derart ist, dass die erste Kartusche (416) derart in das Basiselement (418) eingesetzt ist, dass der Kolben auf der Kolbenstange (450) sitzt, und die zweite Kartusche (480) sich in dem Gehäuse (412) befindet, wobei die durchstechbare Kappe (484) der zweiten Kartusche (480) der ersten Nadel (474) zugewandt ist;
wobei die Fluidtransfervorrichtung **dadurch gekennzeichnet ist, dass** die Kolbenstange (450) fest an der Basisbodenwand gesichert ist und sich von dieser nach oben erstreckt.

2. Fluidtransfervorrichtung nach Anspruch 1, wobei die Nadelnabe (414) eine erste und eine zweite obere Lasche (460, 462) beinhaltet, wobei jede der oberen Laschen einen Vorsprung aufweist, der sich von ihr nach außen erstreckt, wobei die Vorsprünge so angeordnet sind, dass sie in eines der Schlitzpaare (428, 430), die in dem Gehäuse (412) gebildet sind, eingreifen.

3. Fluidtransfervorrichtung nach Anspruch 1, wobei die erste Nadel einen an sie angrenzenden Dorn (218) aufweist, wobei der Dorn (218) und die erste Nadel zwischen sich eine gemeinsame Wand aufweisen.

## Revendications

1. Dispositif de transfert de fluide (410) comprenant :
un boîtier (412) ayant une paroi extérieure (422), une pluralité de paires de fentes (428, 430) formées dans ladite paroi extérieure (422), chacune desdites paires de fentes étant diamétralement opposée l'une à l'autre ;
un moyeu d'aiguille (414) ayant une paroi extérieure (456, 458) et une paroi intérieure (472) s'étendant intérieurement à travers ladite paroi extérieure (456, 458), pour former ainsi des compartiments supérieur et inférieur (492, 494) une première aiguille (474) s'étendant vers le haut à partir de ladite paroi intérieure (472), dans ledit compartiment supérieur (492) une seconde aiguille (476) s'étendant vers le bas à partir de ladite paroi intérieure dans ledit compartiment inférieur (494), ladite paroi intérieure (472) ayant une ouverture à l'intérieur pour former un passage de fluide (478) ;
une première cartouche (416) comportant un piston et un capuchon perçable (490) ;
une seconde cartouche (480) comportant un piston et un capuchon perçable (484) ;
un élément de base (418), ledit élément comportant une paroi inférieure de base (446) et une tige de piston (450) ; et
ledit agencement étant tel que ladite première cartouche (416) est insérée dans ledit élément de base (418) de telle sorte que ledit piston repose sur ladite tige de piston (450), et ladite seconde cartouche (480) est dans ledit boîtier (412) avec ledit capuchon perçable (484) de ladite seconde cartouche (480) faisant face à ladite première aiguille (474) ;
ledit dispositif de transfert de fluide étant **caractérisé en ce que** la tige de piston (450) est fixée de manière fixe à ladite paroi inférieure de base et s'étend vers le haut à partir de celle-ci.

2. Dispositif de transfert de fluide selon la revendication 1, dans lequel ledit moyeu d'aiguille (414) comprend des première et seconde languettes supérieures (460, 462), chacune desdites languettes supérieures comportant une saillie s'étendant vers l'extérieur à partir de celle-ci, lesdites saillies étant agencées pour s'engager avec l'une desdites paires de fentes (428, 430) formées dans ledit boîtier (412).

3. Dispositif de transfert de fluide selon la revendication 1, dans lequel ladite première aiguille comporte une pointe (218) adjacente à celle-ci, ladite pointe (218) et ladite première aiguille ayant une paroi commune entre elles.
